# EUROPÄISCHE PATENTSCHRIFT

(11) **EP 2 396 041 B1**
(45) Veröffentlichungstag und Bekanntmachung des Hinweises auf die Patenterteilung: **05.09.2012**
(21) Anmeldenummer: 11702131.1
(22) Anmeldetag: 13.01.2011
(51) Int. Cl.: A61L 2/04

(54) **VERFAHREN ZUR DESINFEKTION EINER FLASCHE**
METHOD FOR DISINFECTING A BOTTLE
PROCÉDÉ DE DÉSINFECTION D'UNE BOUTEILLE

(30) Priorität: 08.02.2010 AT 1752010
(43) Veröffentlichungstag der Anmeldung: 21.12.2011
(73) Patentinhaber: Mam Babyartikel Gesellschaft m.b.H., 1160 Wien (AT)
(72) Erfinder: RÖHRIG, Peter, A-1160 Wien (AT)
(74) Vertreter: Beetz, Rainer
(86) Internationale Anmeldenummer: PCT/AT2011/000018
(87) Internationale Veröffentlichungsnummer: WO 2011/094773

(56) Entgegenhaltungen:
- WO-A2-2005/041851
- CH-A- 544 547
- US-A- 2 610 755
- US-A- 3 134 495
- US-A- 5 499 729

## Beschreibung

Die Erfindung betrifft ein Verfahren zur Desinfektion einer Flasche, insbesondere Babyflasche, mit einem beidseitig offenen Flaschenmantel, wobei in einer Abgabestellung an einer bodenseitigen Öffnung eine Bodenkappe und an einer saugerseitigen Öffnung ein Sauger mit einem Befestigungsring befestigt sind, wobei der Innenumfang der bodenseitigen Öffnung größer als der Außenumfang des Befestigungsrings ist.

Aus dem Stand der Technik sind bereits verschiedenste Verfahren und Vorrichtungen zum Desinfizieren bzw. zum Sterilisieren von Flaschenkörpern, insbesondere von Babyflaschen mit einem Flaschensauger bekannt. Üblicherweise werden derartige Vorrichtungen Sterilisationsvorrichtungen ("sterilizer") genannt, wobei auch diese Vorrichtungen tatsächlich nur eine Desinfektion (=Keimzahlreduktion um den Faktor 10⁵) und nicht eine Sterilisation (=Keimzahlreduktion um den Faktor 10⁶) durchführen. Nachfolgend werden die beiden Begriffe jedoch synonym verwendet, und es ist hiermit eine Keimzahlreduktion zumindest um den Faktor 10⁵ gemeint.

Zudem sind zahlreiche Babyflaschen bekannt, welche einen beidseitig offenen Flaschenmantel aufweisen, wobei die bodenseitige Öffnung einen größeren Umfang aufweist als die saugerseitige Öffnung. So ist z.B. aus der auf die Anmelderin selbst zurückgehende WO 2005/041851 A2 eine derartige Babyflasche bekannt, wobei hier die vergleichsweise große bodenseitige Öffnung insbesondere dazu vorgesehen ist, um ein großes bodenseitiges Lufteintrittsventil zu ermöglichen. Eine ähnliche Babyflasche mit einer vergleichsweise großen bodenseitigen Öffnung gegenüber der saugerseitigen Öffnung ist aus der DE 87 04 733 U bekannt. In der WO 99/011218 ist eine weitere Babyflasche mit einem beidseitig offenen Mantel beschrieben, auf dem ein Nippelteil bzw. eine Bodenkappe aufgeschraubt ist.

Aus der US 3,134,495 A ist eine weitere Babyflasche mit beidseitig offenem Flaschenmantel bekannt; an der saugerseitigen Öffnung des Flaschenmantels ist ein Sauger mittels eines Befestigungsringsangeordnet. Die Flasche weist zudem ein bodenseitiges Luftventil auf, welches durch einen weiteren Sauger gebildet ist, der ident zum Trink-Sauger ausgeführt ist. Der Luftventil-Sauger ist über einen Flansch zwischen dem bodenseitigen offenen Ende des Flaschenmantels und einem damit verschraubten Befestigungsring eingeklemmt. Der Sauger fungiert gemeinsam mit einem in einer Bodenplatte des Befestigungsrings angeordneten Lufteintrittsöffnung als Luftventil, indem Luft durch die Öffnung in den Hohlraum des Saugers einströmen und von dort über eine schlitzförmige Öffnung des Saugers ins Flascheninnere gelangen kann.

Üblicherweise werden derartige Babyflaschen in speziell hierfür vorgesehenen Vorrichtungen sterilisiert bzw. desinfiziert.

Aus der DE 31 49 754 A ist beispielsweise eine Sterilisations- bzw. Desinfektionsvorrichtung bekannt, bei welcher mehrere Aufnahmevorrichtungen für Babyflaschen sowie gesonderte Aufnahmevorrichtungen für Flaschensauger vorgesehen sind. Nachteilig ist hierbei insbesondere, dass bei dieser Vorrichtung nur eine einzige, vergleichsweise große Desinfektionskammer vorgesehen ist, so dass die Flaschen nach dem Desinfektionsvorgang nur teilweise desinfiziert sind bzw. ein sehr inhomogenes Desinfektionsniveau erzielt wird. Zudem sind die Anschaffungskosten relativ hoch und derartige Vorrichtungen benötigen auch einen relativ großen Platzbedarf.

Aus der GB 2 395 108 A ist eine weitere Sterilisations-Vorrichtung zum Reinigen einer Babyflasche bekannt, welche die im Zusammenhang mit der DE 31 49 754 A beschriebenen Nachteile aufweist. Die Vorrichtung weist einen Behälter auf, in dem ein Wasserbad aufgenommen ist. Die Elemente der Babyflasche werden in das Wasserbad eingelegt, das mittels eines Schallgebers zu Vibrationen angeregt werden kann. Zudem können die zu reinigenden Elemente einer UV-Strahlung ausgesetzt werden.

Eine zu Desinfektionszwecken speziell ausgebildete Babyflasche ist aus der GB 2 324 788 A bekannt. Hierin ist eine Babyflasche gezeigt, bei welcher ein Sauger, ein Befestigungsring sowie eine Abdeckkappe in einer Nahrungsabgabestellung sowie in einer Desinfektionsstellung angeordnet werden können. Hierbei handelt es sich jedoch um einen bodenseitig geschlossenen Flaschenkörper, so dass der Sauger sowie der Befestigungsring lediglich in einer von der Abdeckkappe geschlossenen Kammer zu Desinfektionszwecken aufgenommen werden können. Um den Sauger in einer Desinfektionsstellung in einer von dem Flaschenkörper beabstandeten Stellung halten zu können, sind nachteiligerweise verschiedene speziell ausgebildete Verstau- bzw. Haltemittel erforderlich.

In der FR 2 769 841 A1 ist eine weitere bodenseitig geschlossene Babyflasche beschrieben, welche zur Desinfizierung des Saugers mit einer Verschlusskappe verbunden wird, die auf den Behältermantel aufgeschraubt wird. Im Inneren der Verschlusskappe ist eine Desinfektionskammer für den Sauger ausgebildet.

Ziel der vorliegenden Erfindung hingegen ist es, ein energie-und zeitsparendes Verfahren zur Desinfektion von Flaschen der eingangs angeführten Art bzw. eine besondere Verwendung derartiger Flaschen zu schaffen, bei welcher weder gesonderte zur Desinfektion ausgebildete Vorrichtungen erforderlich sind noch spezielle Haltemittel zur Positionierung des Saugers in einer vom Flaschenkörper beabstandeten Desinfektionsstellung.

Erfindungsgemäß wird dies dadurch erzielt, dass zum Überführen in eine Desinfektionsstellung die Bodenkappe vom-Flaschenmantel abgenommen wird, der Sauger und der Befestigungsring auf die Bodenkappe aufgesetzt werden bzw. in den Flaschenmantel eingeführt werden, die Bodenkappe an der bodenseitigen Öffnung angebracht wird, ein Desinfektionsmittel, insbesondere Wasser, in den Flaschenmantel eingefüllt wird, bevor die Flasche zwecks Desinfektion erwärmt wird. Überraschenderweise hat sich gezeigt, dass - sofern die üblicherweise kreisrunde bodenseitige Öffnung des Flaschenkörpers größer ist als der üblicherweise im Wesentlichen kreisrunde Außenumfang des Befestigungsrings - nach Abnahme der Bodenkappe von dem Flaschenkörper sowohl der Sauger als auch der Befestigungsring im Inneren des Flaschenmantels - vorzugsweise nachdem diese beiden Teile auf die Bodenkappe aufgesetzt wurden - aufgenommen werden können. Somit ergibt sich im Inneren des Flaschenmantels, nachdem die Bodenkappe wieder an der bodenseitigen Öffnung befestigt wurde, eine Art Desinfektionskammer, so dass nachdem ein Desinfektionsmittel, üblicherweise Wasser, in die Desinfektionskammer eingefüllt wurde, dieses mittels Erwärmung zum Sieden bzw. Verdampfen gebracht werden kann, wodurch alle Teile der Flasche, d.h. insbesondere der Flaschenmantel, die Bodenkappe, der Befestigungsring sowie der Sauger auf einfachste Weise zuverlässig desinfiziert werden können. Hierfür ist weder eine besonders für Desinfektionszwecke ausgestaltete Vorrichtung noch zusätzliche Haltemittel oder dgl. erforderlich. Somit ergibt sich für den Benutzer ein äußerst einfach handhabbares Verfahren zur Desinfektion von Flaschen bzw. eine neuartige Verwendung von an sich bereits bekannten Babyflaschen. Zudem ist das Verfahren vergleichsweise energie- und zeitsparend, da lediglich die Flächen sterilisiert werden, die mit Nahrung oder dem Kindermund in Berührung kommen. Demzufolge werden insbesondere die Innenflächen der Flaschenteile (Flaschenmantel, Bodenkappe, und Befestigungsring), sowie der gesamte Sauger, d.h. Innenfläche und Mundanlageflächen mit einer vergleichsweise geringen Menge Desinfektionsmittel sterilisiert. Eine aus hygienischer Sicht nicht erforderliche aber äußerst energieintensive Desinfektion der Außenflächen kann somit unterbleiben.

Insbesondere ist es vorteilhaft, wenn der Befestigungsring samt Sauger als Einheit auf die Bodenkappe aufgesetzt wird bzw. in den Flaschenmantel eingeführt wird. Für eine gute, genaue Positionierung des Befestigungsrings können auf der Bodenkappe selbst oder auf einer etwaigen Membran einer Ventilvorrichtung Halterippen bzw. -stege zur Positionierung bzw. Befestigung des Rings bzw. des Saugers vorgesehen sein. Um auch eine zuverlässige Desinfektion des unteren Randbereichs des Befestigungsrings zu erzielen, ist es günstig wenn der untere Randbereich möglichst freiliegend in der Bodenfläche aufgenommen wird. Dies wird auf einfache und zuverlässige Weise erzielt, wenn die Membran eine im Wesentlichen zylindrische Mantelfläche aufweist, an deren Innenseite mehrere, vorzugsweise drei, über den Umfang verteilt angeordnete vorzugsweise im Wesentlichen in radialer Richtung ins Innere vorspringende Haltestege vorgesehen sind. Hiedurch kann der Befestigungsring auf sichere und einfache Weise auf den vorspringenden Haltestegen der Membran beabstandet von der Bodenfläche positioniert werden, so dass vorteilhafterweise nur kleine Kontaktflächen im Bereich der Haltestege zwischen der Membran und dem Befestigungsring erzeugt werden.

Sofern der Sauger gemeinsam mit dem Befestigungsring als Einheit auf die Bodenkappe aufgesetzt bzw. im Flaschenmantel eingeführt wird, ist es vorteilhafterweise nicht erforderlich, nach Ende der Desinfektion den Sauger durch die Öffnung im Befestigungsring durchzuziehen. Bei herkömmlichen Desinfektionsvorrichtungen, bei welchen diese beiden Teile getrennt von einander desinfiziert werden, wird der Flaschensauger üblicherweise im Nippelbereich erfasst und durch die Öffnung im Befestigungsring durchgezogen, bevor der Befestigungsring auf den Flaschenmantel bzw. -körper aufgeschraubt wird. Durch dieses Erfassen des zuvor desinfizierten Saugers wird nachteiligerweise die Keimzahl wieder wesentlich erhöht, so dass ein Kleinkind bzw. ein Baby, welches mit der Flasche gefüttert wird, gerade im Lippen- bzw. Zungenanlagenbereich mit den Flächen, die eine erhöhte Keimzahl aufweisen, in Berührung kommt. Sofern jedoch der - üblicherweise in der Öffnung des Befestigungsring klemmend gehaltene - Sauger samt dem Befestigungsring als Einheit desinfiziert wird, kann nach Ende des Desinfektionsvorgangs der Befestigungsring lediglich an seiner äußeren Schürze erfasst werden und der Sauger somit, ohne dass der Benutzer mit dem Sauger in Kontakt kommt, vor einer Verfütterung auf der saugerseitigen Öffnung befestigt werden. Um eine neuerliche Kontaminierung zu vermeiden, wird bei bekannten Desinfektionsvorrichtung häufig eine Zange als Zusatzteil hinzugefügt, die zur Saugermontage verwendet werden soll. Derartige Zusatzteile gehen aber nachteiligerweise häufig verloren, oder werden zu Hause vergessen und stehen sodann unterwegs nicht zur Verfügung. Bei der erfindungsgemäßen Lösung ist hingegen ein derartiger Zusatzteil nicht erforderlich, so dass zuverlässig immer alle zur Desinfektion und zum verschmutzungsfreien Zusammenbau der Flasche benötigten Teile vorhanden sind. Zudem können durch die Vermeidung von Zusatzteilen die Kosten gesenkt werden.

Gemäß dem erfindungsgemäßen Verfahren werden vorteilhafterweise grundsätzlich nach dem Sterilisieren die verschiedenen Flaschenteile nur an jenen Flächen angegriffen, deren Sterilität für den Füttervorgang nicht erforderlich ist. Tests haben gezeigt, dass - sofern Befestigungsring und Sauger nicht voneinander getrennt werden - auch jene Bereiche, wo die Oberseite eines Flansches des Saugers im Wesentlichen an der Unterseite des Befestigungsrings anliegt, ausreichend desinfiziert werden.

Üblicherweise weisen Babyflaschen auch eine Abdeckkappe auf, um den Sauger beim Transport vor Verschmutzung zu schützen bzw. einen ungewollten Flüssigkeitsaustritt zu verhindern. Um auch derartige Abdeckkappen ausreichend zu desinfizieren, ist es vorteilhaft, wenn eine Abdeckkappe vorgesehen ist, die in einer Aufbewahrungsstellung auf dem Befestigungsring aufgesetzt ist, in der Abgabestellung vom Befestigungsring abgenommen ist und in der Desinfektionsstellung auf die saugerseitige Öffnung aufgesetzt wird. Somit kann die Abdeckkappe vorteilhafterweise als eine Art Deckel zur Ausbildung einer Desinfektionskammer auf den Flaschenmantel aufgesetzt werden und ebenfalls zuverlässig desinfiziert werden.

Sofern die Abdeckkappe innenseitige Arretierungselemente, insbesondere eine Rastnut oder -elemente aufweist, kann die Abdeckkappe vorteilhafterweise auf dem Flaschenmantel - ohne die obere Öffnung zu verschließen - fixiert werden, so dass die Flasche nach dem Sterilisieren ungeöffnet steril aufbewahrt bzw. transportiert werden kann, da alle hinsichtlich einer Kontaminierung kritischen Flächen von außen nicht zugänglich sind.

Hinsichtlich einer einfachen Bemessung einer zweckmäßigen Menge an Desinfektionsmittel ist es günstig, wenn die Abdeckkappe zur Bemessung des Desinfektionsmittels verwendet wird. Die Abdeckkappe kann hierzu ein abgesetztes Flüssigkeitsreservoir zur Aufnahme der entsprechenden Menge an Desinfektionsmittel oder z.B. Maßlinien in der Art eines Messbechers aufweisen.

Um eine ausreichende Desinfektion der einzelnen Teile der Flasche zu erzielen, ist es günstig, wenn zwischen 10 und 40 ml Desinfektionsmittel in den Flaschenmantel eingefüllt werden. Hierbei ist es vorteilhaft, wenn eine Unterkante des Befestigungsrings, insbesondere mittels Haltestegen, oberhalb des Spiegels des Desinfektionsmittels nach Einfüllen in den Flaschenmantel angeordnet ist. Wenn die Flasche auf eine Temperatur zwischen 100°C und 120°C erwärmt wird, ist zuverlässig gewährleistet, dass das Desinfektionsmittel, üblicherweise Wasser, das in die Flasche eingefüllt wurde, siedet bzw. verdampft und somit die gewünschte Desinfektion erzielt wird. Als vorteilhaft hat sich hierbei erwiesen, wenn - abhängig von Erhitzungstemperatur - die Flasche für einen Zeitraum zwischen 5 s und 3 min erwärmt wird.

Ein Verdampfen bzw. Sieden ist auf einfache Weise binnen kürzester Zeit sichergestellt, wenn die Flasche in einem Mikrowellenherd erwärmt wird.

Die Erfindung wird nachstehend anhand eines in den Zeichnungen dargestellten bevorzugten Ausführungsbeispiels, auf das sie jedoch keinesfalls beschränkt sein soll, noch näher erläutert. Im Einzelnen zeigen in den Zeichnungsunterlagen:
Fig. 1 eine Schnittansicht einer an sich bekannten Babyflasche, bei welcher der Innenumfang der bodenseitigen Öffnung größer ist als der Außenumfang eines Befestigungsrings;
Fig. 2 eine Schnittansicht der Babyflasche gemäß Fig. 1 in einer Zwischenstellung zwischen einer Abgabe- und einer Desinfektionsstellung bei welcher ein Sauger samt Befestigungsring sowie eine Bodenkappe vom Flaschenmantel abgeschraubt wurden;
Fig. 2a eine perspektivische Ansicht einer in der Bodenkappe eingesetzten Membran;
Fig. 3 eine Schnittansicht der Babyflasche gemäß den Fig. 1 und 2 beim Befüllen einer vom Flaschenmantel und der Bodenkappe gebildeten Desinfektionskammer mit Wasser; und
Fig. 4 eine Schnittansicht der Babyflasche in einer Desinfektionsstellung.

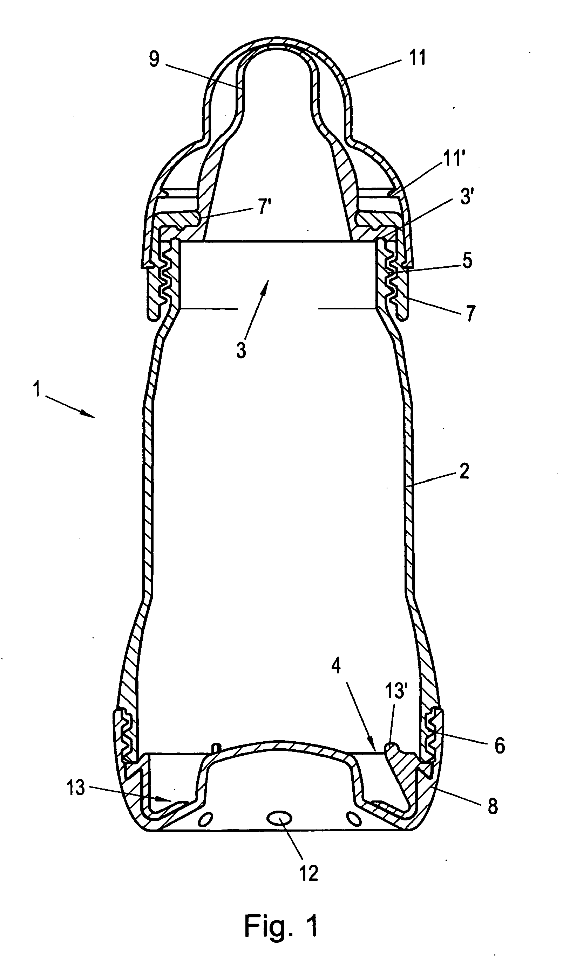

In Fig. 1 ist eine Babyflasche 1 gezeigt, welche einen beidseitig offenen Flaschenmantel 2 mit einer saugerseitigen Öffnung 3 und einer bodenseitigen Öffnung 4 aufweist. In den den Öffnungen 3, 4 benachbarten beiden Endbereichen des Flaschenmantels 2 ist jeweils ein Außengewinde 5, 6 vorgesehen, über welches auf einfache Weise ein Befestigungsring bzw. -körper 7 bzw. eine Bodenkappe 8, die jeweils ein entsprechendes Innengewinde aufweisen, befestigt werden können. Der Befestigungsring 7 ist zur klemmenden Befestigung eines Saugers 9 vorgesehen, wobei der Sauger 9 im unteren Bereich seines Schaftes klemmend in einer Öffnung 7' des Befestigungsrings 7 gehalten wird und ein Flansch des Saugers 9 zwischen einem oberen Rand 3' des Flaschenmantels 2 und dem Befestigungsring 7 eingeklemmt ist. Zudem weist die Flasche 1 eine Abdeckkappe 11 auf, welche auf dem Befestigungsring 7 mittels einer Schnappverbindung befestigt ist.

Die Bodenkappe 8 weist bei dem gezeigten Ausführungsbeispiel Lufteintrittsöffnungen 12 auf, die mit einer Membran 13 abgedeckt sind, um einen bodenseitigen Lufteintritt ins Flascheninnere zu ermöglichen. Für das erfindungsgemäße Verfahren ist dieses Bodenventil jedoch keinesfalls erforderlich. Es kann selbstverständlich lediglich eine herkömmliche Bodenkappe 8 ohne jegliche Ventilvorrichtung vorgesehen sein.

In Fig. 1 ist somit die Flasche 1 im Wesentlichen in ihrer Aufbewahrungsstellung gezeigt, wobei zum Überführen in die Abgabestellung, in welcher eine in der Flasche 1 aufgenommenen Flüssigkeit abgegeben werden kann, lediglich die vorherige Abnahme der Abdeckkappe 11 erforderlich ist. Gemäß dem erfindungsgemäßen Verfahren kann eine derartige Flasche 1 auf einfacher Weise zu Desinfektionszwecken in eine Desinfektionsstellung überführt werden.

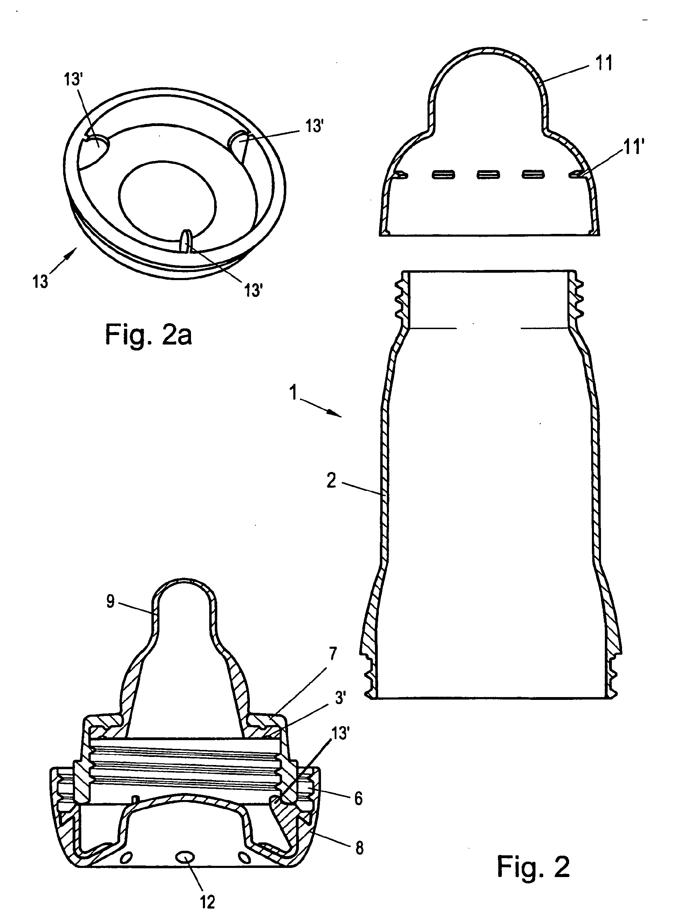

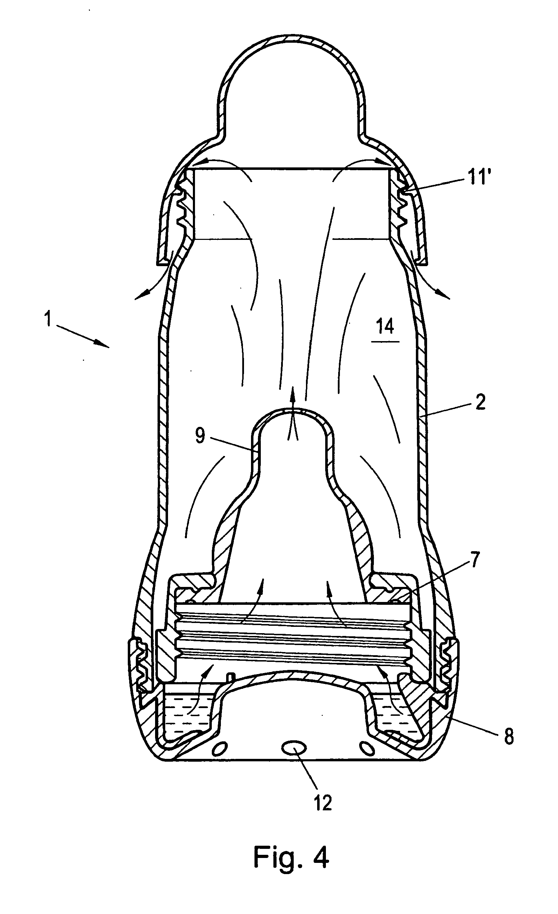

In Fig. 2 ist eine Zwischenstellung zwischen der in Fig. 1 gezeigten Aufbewahrungsstellung und der in Fig. 4 gezeigten Desinfektionsstellung gezeigt. Hierbei wurde zunächst die Abdeckkappe 11 vom Befestigungsring 7 abgenommen und sodann der Befestigungsring 7 samt dem in der Öffnung 7' des Befestigungsrings 7 klemmend aufgenommenen Sauger 9 von der saugerseitigen Öffnung 3 des Flaschenmantels 2 abgeschraubt. Bevor oder nachdem der Befestigungsring 7 samt Sauger 9 vom Flaschenmantel 2 abgeschraubt wird, wird auch die Bodenkappe 8 vom Flaschenmantel 2 abgeschraubt, so dass der Befestigungsring 7 samt dem hierin klemmend aufgenommenen Sauger 9 auf die Bodenkappe 8 bzw. einer in der Bodenkappe 8 aufgenommenen Membran 13 aufgesetzt werden kann. Hierbei wird der Befestigungsring 7 auf die Haltestege 13' der Membran 13 aufgesetzt, die in Fig. 2a im Detail dargestellt sind. Hierbei ist ersichtlich, dass die Membran 13 eine im Wesentlichen zylindrische Mantelfläche aufweist, an deren Innenseite drei um 120° zueinander versetzt angeordnete ins Innere vorspringende Haltestege 13' vorgesehen sind. Auf diesen Haltestegen 13' kann der Befestigungsring 7 somit auf einfache und sichere Weise beabstandet von der Bodenfläche der Membran 13 angeordnet werden. Anschließend wird die Bodenkappe 8 samt dem hierin aufgesetzten Befestigungsring mit dem Sauger 9 wiederum auf dem Flaschenmantel 2 aufgeschraubt, so dass von dem Flaschenmantel 2 und der Bodenkappe 8 eine Desinfektionskammer 14 gebildet wird.

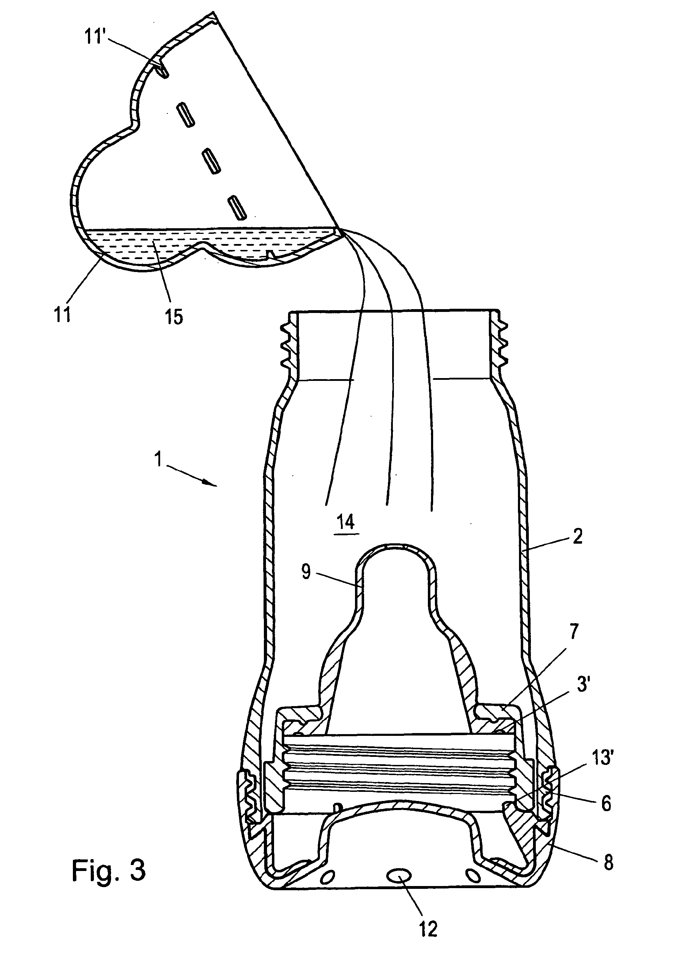

Wie in Fig. 3 ersichtlich wird in die Desinfektionskammer 14 sodann vorzugsweise mit Hilfe der Abdeckkappe 11, welche in ihrer umgedrehten Stellung eine Art Messbecher bildet, Wasser 15 zu Desinfektionszwecken in die Desinfektionskammer 14 eingefüllt. Die Abdeckkappe 11 weist hierbei vorzugsweise einen gegenüber der übrigen Abdeckkappe 11 abgesetzten Abschnitt 11' auf, welcher ein Aufnahmevolumen von ca. 10 bis 40ml aufweist; dies stellt eine zweckmäßige Menge Wasser zur Desinfektion der Flasche 1 dar. Somit kann die Abdeckkappe 11 auf einfache Weise als eine Art Messbecher zur entsprechenden Bemessung des in die Desinfektionskammer 14 eingefüllten Wassers dienen.

Um zugleich auch eine Desinfektion der Abdeckkappe 11 zu erzielen, wird diese - wie in Fig.4 ersichtlich - abschließend lose auf die saugerseitige Öffnung 3 des Flaschenmantels 2 aufgesetzt. Wie ebenfalls in Fig.4 ersichtlich wird der Befestigungsring 7 mit Hilfe der Haltestege 13' derart in Position gehalten, dass - nachdem die bevorzugte Menge an Desinfektionsmittel eingebracht wurde - die Unterkante des Befestigungsrings 7 oberhalb des Spiegels des Desinfektionsmittels angeordnet ist; somit ist auch eine zuverlässige Desinfektion des unteren Randbereichs des Befestigungsrings 7 gewährleistet.

In dieser Desinfektionsstellung wird die Flasche 1 sodann einer Wärmequelle, z.B. einem Mikrowellenherd, zugeführt. Hierbei kann die Abdeckkappe 11 innenseitig Arretierungelemente 11', insbesondere Rastelemente aufweisen, so dass die Flasche nach dem Sterilisieren ungeöffnet steril aufbewahrt bzw. transportiert werden kann, da alle hinsichtlich einer Kontaminierung kritischen Flächen von außen nicht zugänglich sind.

Für die Desinfektion von Babyflaschen hat sich ein Desinfektionsniveau von A₀=600 entsprechend den Normvorschriften als zweckmäßig herausgestellt. Um dieses Desinfektionsniveau zu erzielen reicht bei einer Oberflächentemperatur von 100°C eine Einwirkdauer von 6s aus; bei niedrigeren Temperaturen ist eine entsprechend höhere Einwirkzeit vorzusehen. Bei Verwendung von 20ml Wasser zur Desinfektion einer Babyflasche wird dieses Desinfektionsniveau mit bei einer Mikrowellenleistung von 800W innerhalb von 2 Minuten erzielt. Mit Hilfe des in der Desinfektionskammer 14 aufsteigenden Dampfes werden somit sowohl die die Dampfkammer 14 bildende Bodenkappe 8 und der Flaschenmantel 2 als auch der darin aufgenommene Sauger 9 und der Befestigungsring 7 ebenso wie die Abdeckkappe 11 zuverlässig desinfiziert.

Um nach der Desinfektion die Flasche 1 wiederum in die in Fig. 1 gezeigte Aufbewahrungsstellung bzw. die Abgabestellung überzuführen, wird wiederum die Bodenkappe 8 vom Flaschenmantel 2 abgeschraubt, sodann der Befestigungsring 7 samt dem hierin aufgenommenen Sauger 9 auf der saugerseitigen Öffnung 3 des Flaschenmantels 2 aufgeschraubt und anschließend (oder davor) wiederum die Bodenkappe 8 auf der bodenseitigen Öffnung 4 aufgeschraubt.

Sofern der Befestigungsring 7 gemeinsam mit dem Sauger 9 als gemeinsame Einheit in die Dampfkammer 14 eingesetzt wurde, besteht ein wesentlicher Vorteil des erfindungsgemäßen Verfahrens darin, dass bei dem Zusammenstellen der Flasche 1 in die Abgabestellung der Sauger 9 nach der Desinfektion nicht vom Benutzer berührt werden muss. Vielmehr kann der Befestigungsring 7 lediglich an seiner zylindrischen Schürze erfasst werden und somit der Sauger 9 ohne jeglichen Kontakt mit einer nicht sterilen Hand oder dgl. auf dem Flaschenmantel 2 befestigt werden. Bei herkömmlichen Desinfektionsverfahren hingegen ist es erforderlich, den Sauger 9 durch die Öffnung 7' im Befestigungsring 7 hindurch zu ziehen, wobei üblicherweise der Sauger 9 im Bereich des Nippels, d.h. im Bereich der Zungen- und Lippenanlage des Babys bzw. Kleinkindes, mit nicht sterilen Händen erfasst wird und somit wesentliche Abschnitte der zuvor desinfizierten Teile wiederum verschmutzt werden. Dies kann durch das erfindungsgemäße Verfahren zuverlässig verhindert werden.

## Patentansprüche

1. Verfahren zur Desinfektion einer Flasche (1), insbesondere Babyflasche, mit einem beidseitig offenen Flaschenmantel (2), wobei in einer Abgabestellung an einer bodenseitigen Öffnung (4) eine Bodenkappe (8) und an einer saugerseitigen Öffnung (3) ein Sauger (9) mit einem Befestigungsring (7) befestigt sind, wobei der Innenumfang der bodenseitigen Öffnung (4) größer als der Außenumfang des Befestigungsrings (7) ist, **dadurch gekennzeichnet, dass** zum Überführen in eine Desinfektionsstellung die Bodenkappe (8) vom Flaschenmantel (2) abgenommen wird, der Sauger (9) und der Befestigungsring (7) auf die Bodenkappe (8) aufgesetzt werden bzw. in den Flaschenmantel (2) eingeführt werden, die Bodenkappe (8) an der bodenseitigen Öffnung (4) angebracht wird, ein Desinfektionsmittel, insbesondere Wasser (15), in den Flaschenmantel (2) eingefüllt wird, bevor die Flasche (1) zwecks Desinfektion erwärmt wird.

2. Verfahren nach Anspruch 1, **dadurch gekennzeichnet, dass** der Befestigungsring (7) samt Sauger (9) als Einheit auf die Bodenkappe (8) aufgesetzt wird bzw. in den Flaschenmantel (2) eingeführt wird.

3. Verfahren nach Anspruch 2, **dadurch gekennzeichnet, dass** der Befestigungsring (7) auf der Bodenkappe (8) bzw. einer Membran (13) mittels zumindest einer Halterippe bzw. -steg (13') positioniert gehaltert wird.

4. Verfahren nach Anspruch 3, **dadurch gekennzeichnet, dass** die Membran (13) eine zylindrische Mantelfläche aufweist, an deren Innenseite mehrere, über den Umfang verteilt angeordnete, in radialer Richtung ins Innere vorspringende Haltestege (13') vorgesehen sind.

5. Verfahren nach einem der Ansprüche 1 bis 4, **dadurch gekennzeichnet, dass** eine Abdeckkappe (11) vorgesehen ist, die in einer Aufbewahrungsstellung auf dem Befestigungsring (7) aufgesetzt ist, in der Abgabestellung vom Befestigungsring (7) abgenommen ist und in der Desinfektionsstellung auf die saugerseitige Öffnung (3) aufgesetzt wird.

6. Verfahren nach Anspruch 5, dadurch gekennezeichnet, dass die Abdeckkappe (11) mittels innenseitiger Arretierungselemente (11'), insbesondere einer Rastnut oder -elementen, auf dem Flaschenmantel (2) befestigt wird.

7. Verfahren nach einem der Ansprüche 5 oder 6, **dadurch gekennzeichnet, dass** die Abdeckkappe (11) zur Bemessung des Desinfektionsmittels verwendet wird.

8. Verfahren nach einem der Ansprüche 1 bis 7, **dadurch gekennzeichnet, dass** zwischen 10 und 40 ml Desinfektionsmittel in den Flaschenmantel (2) eingefüllt werden.

9. Verfahren nach einem der Ansprüche 1 bis 8, **dadurch gekennzeichnet, dass** die Flasche (1) auf eine Temperatur zwischen 100 °C und 120°C erwärmt wird.

10. Verfahren nach einem der Ansprüche 1 bis 9, **dadurch gekennzeichnet, dass** die Flasche (1) in einem Mikrowellenherd erwärmt wird.

## Claims

1. A method for disinfecting a bottle (1), in particular a baby bottle, including a bottle jacket (2) which is open on both ends, wherein, in a dispensing position, a bottom cap (8) is fastened to a bottom-side opening (4) and a teat (9) is fastened to a teat-side opening (3) by a fastening ring (7), the inner periphery of the bottom-side opening (4) being larger than the outer periphery of the fastening ring (7), **characterized in that**, for transfer into a disinfection position, the bottom cap (8) is removed from the bottle jacket (2), the teat (9) and the fastening ring (7) are placed onto the bottom cap (8) and introduced into the bottle jacket (2) respectively, the bottom cap (8) is attached to the bottom-side opening (4), a disinfectant, particularly water (15), is filled into the bottle jacket (2) before the bottle (1) is heated for disinfection.

2. A method according to claim 1, **characterized in that** the fastening ring (7) along with the teat (9) are placed onto the bottom cap (8), and introduced into the bottle jacket (2) respectively, as a unit.

3. A method according to claim 2, **characterized in that** the fastening ring (7) is held in its position on the bottom cap (8), or a membrane (13), by at least one holding rib or holding web (13').

4. A method according to claim 3, **characterized in that** the membrane (13) has a cylindrical lateral surface on whose inner side several holding webs (13') are provided in a manner distributedly arranged about its periphery and protruding into the interior in a radial direction.

5. A method according to any one of claims 1 to 4, **characterized in that** a cover cap (11) is provided, which is placed on the fastening ring (7) in a storage position, removed from the fastening ring (7) in the dispensing position, and placed on the teat-side opening (3) in the disinfection position.

6. A method according to claim 5, **characterized in that** the cover cap (11) is fixed to the bottle jacket (2) by internal locking elements (11'), in particular, a snap-in groove or snap-in elements.

7. A method according to any one of claims 5 or 6, **characterized in that** the cover cap (11) is used for measuring the disinfectant.

8. A method according to any one of claims 1 to 7, **characterized in that** between 10 and 40 ml of disinfectant are filled into the bottle jacket (2).

9. A method according to any one of claims 1 to 8, **characterized in that** the bottle (1) is heated to a temperature between 100°C and 120°C.

10. A method according to any one of claims 1 to 9, **characterized in that** the bottle (1) is heated in a microwave oven.

## Revendications

1. Procédé pour désinfecter une bouteille (1), en particulier un biberon, comprenant une enveloppe de bouteille (2) ouverte des deux côtés, un capuchon de fond (8) étant fixé dans une position de distribution sur une ouverture (4) côté fond et une tétine (9) avec une bague de fixation (7) étant fixée sur une ouverture (3) côté tétine, le pourtour intérieur de l'ouverture (4) côté fond étant supérieur au contour extérieur de la bague de fixation (7), **caractérisé en ce que** le capuchon du fond (8) est enlevé de l'enveloppe de bouteille (2) pour le transfert dans une position de désinfection, la tétine (9) et la bague de fixation (7) sont posées sur le capuchon de fond (8) ou sont introduites dans l'enveloppe de bouteille (2), le capuchon de fond (8) est placé sur l'ouverture (4) côté fond, un désinfectant, en particulier de l'eau (15), est introduit dans l'enveloppe de bouteille (2) avant que la bouteille (1) soit réchauffée pour la désinfection.

2. Procédé selon la revendication 1, **caractérisé en ce que** la bague de fixation (7) avec la tétine (9) est posée comme unité sur le capuchon de fond (8) ou est introduite dans l'enveloppe de bouteille (2).

3. Procédé selon la revendication 2, **caractérisé en ce que** la bague de fixation (7) est maintenue positionnée sur le capuchon de fond (8) ou une membrane (13) au moyen d'au moins une rainure ou d'une barrette de retenue (13').

4. Procédé selon la revendication 3, **caractérisé en ce que** la membrane (13) présente une surface d'enveloppe cylindrique, sur le côté intérieur de laquelle sont prévues plusieurs barrettes de retenue (13') disposées et réparties sur le pourtour et avançant dans le sens radial vers l'intérieur.

5. Procédé selon l'une des revendications 1 à 4, **caractérisé en ce qu'**il est prévu un capuchon de recouvrement (11), qui est posé dans une position de conservation sur la bague de fixation (7), est enlevé de la bague de fixation (7) dans la position de distribution et est posé sur l'ouverture (3) côté tétine dans la position de désinfection.

6. Procédé selon la revendication 5, **caractérisé en ce que** le capuchon de recouvrement (11) est fixé au moyen d'éléments d'arrêt (11') côté intérieur, en particulier une rainure ou des éléments d'arrêt, sur l'enveloppe de bouteille (2).

7. Procédé selon l'une des revendications 5 et 6, **caractérisé en ce que** le capuchon de recouvrement est utilisé pour le mesurage du désinfectant.

8. Procédé selon l'une des revendications 1 à 7, **caractérisé en ce que** entre 10 et 40 ml de désinfectant sont versés dans l'enveloppe de bouteille (2).

9. Procédé selon l'une des revendications 1 à 8, **caractérisé en ce que** la bouteille (1) est réchauffée à une température comprise entre 100°C et 120°C.

10. Procédé selon l'une des revendications 1 à 9, **caractérisé en ce que** la bouteille (1) est réchauffée dans un four à microondes.
